# EUROPÄISCHE PATENTSCHRIFT

(11) **EP 0 804 610 B1**
(45) Veröffentlichungstag und Bekanntmachung des Hinweises auf die Patenterteilung: **03.11.1999**
(21) Anmeldenummer: 96939856.9
(22) Anmeldetag: 20.11.1996
(51) Int. Cl.: C12Q 1/32

(54) **STABILISIERTE COENZYM-LÖSUNGEN UND DEREN VERWENDUNG ZUR BESTIMMUNG VON DEHYDROGENASEN BZW. DEREN SUBSTRATE IM ALKALISCHEN MILIEU**
STABILISED COENZYME SOLUTIONS AND THE USE THEREOF FOR THE DETERMINATION OF DEHYDROGENASES OR THE SUBSTRATES THEREOF IN AN ALKALINE MEDIUM
SOLUTIONS DE COENZYMES STABILISEES ET LEUR UTILISATION POUR LA DETERMINATION DE DESHYDROGENASES OU DE LEURS SUBSTRATS EN MILIEU ALCALIN

(30) Priorität: 22.11.1995 DE 19543493
(43) Veröffentlichungstag der Anmeldung: 05.11.1997
(73) Patentinhaber: Roche Diagnostics GmbH, 68298 Mannheim (DE)
(72) Erfinder: ZIELENSKI, Ralf, D-83673 Bichl (DE)
(86) Internationale Anmeldenummer: EP9605107
(87) Internationale Veröffentlichungsnummer: WO9719190

(56) Entgegenhaltungen:
- EP-A- 0 096 288
- EP-A- 0 253 520
- CH-A- 639 398
- US-A- 3 700 654
- US-A- 4 310 624
- EUR. J. BIOCHEM. (1980), 110(2), 465-73 CODEN: EJBCAI;ISSN: 0014-2956, 1980, XP000646607 KUCHEL, PHILIP W. ET AL: "Determination of the stability constants of manganese (2+) and magnesium (2+) ion complexes of the components of the NADP -linked isocitrate dehydrogenase reaction by electron spin resonance"
- CAN. J. BIOCHEM. (1979), 57(7), 995-9 CODEN: CJBIAE;ISSN: 0008-4018, 1979, XP000646606 GREEN, M. KIRK ET AL: "The stoichiometry and stability of the NADP complexes with manganese (II) ions as studied by electron paramagnetic resonance"

## Beschreibung

Die Erfindung betrifft stabilisierte wässrige Lösungen eines Coenzyms für wasserstoffübertragende Enzyme sowie deren Verwendung zur Bestimmung eines entsprechenden Analyten (Substrates) in reduzierter Form oder der Enzymaktivität einer entsprechenden Dehydrogenase. Die stabilisierte Lösung enthält ein Schwermetallsalz und gegebenenfalls als weitere Komponente einen Komplexbildner und ermöglicht dadurch die Bestimmung im alkalischen Milieu, bevorzugt bei einem pH-Wert von 8,5 bis 10,0.

Die Bestimmung von Enzymaktivitäten (od. Substratkonzentrationen), insbesondere in Blut-Serum oder -Plasma, spielt eine wichtige Rolle in der klinisch chemischen Diagnostik. Häufig werden dazu Testverfahren verwendet, die auf der Reduktion von Nicotinamid-adenin-dinucleotid ("NAD") bzw. Nicotinamid-adenin-dinucleotid-phosphat ("NADP") und der photometrischen Erfassung der dabei erfolgenden Änderung des Absorptionsverhaltens im ultravioletten Wellenlängenbereich (λ=334, 340 oder 365 nm) beruhen. Bei der Wahl geeigneter Testbedingungen ist diese Änderung linear proportional zu der zu bestimmenden Enzymaktivität (bzw. Substratkonzentration).

Zur Bestimmung der Enzymaktivität von beispielsweise Lactatdehydrogenase (LDH, E.C.1.1.1.27) wird heute allgemein das in Eur. J. Chin. Chem. Chin. Biochem. 31, 897 (1994) und Eur. J. Chin. Chem. Chin. Biochem. 32, 639 (1994) beschriebene Verfahren empfohlen. Das Testprinzip sieht die Oxidation von Lactat zu Pyruvat unter gleichzeitiger Reduktion eines Coenzyms wie NAD oder NADP zu NADH bzw. NADPH vor. Eine solche - wie hier beispielsweise von LDH katalysierte - Umsetzung findet im alkalischen Medium (pH 9,4) statt, d.h. unter Bedingungen, unter denen NAD bzw. NADP bekanntermaßen instabil ist. Diese Instabilität äußert sich in einem verhältnismäßig raschen Extinktionsanstieg (dem sog. Reagenzleerwert) im, die Messung betreffenden Wellenlängenbereich, so daß bereits nach kurzer Zeit (3 Monate) auch bei Kühllagerung (2° bis 8°C) die Reagenzkombination unbrauchbar wird. Dieses Problem stellt sich im besonderen Maße für die Herstellung gebrauchsfertiger, langzeitstabiler Flüssigreagenzien, die dem Anwender eine möglichst einfache und sichere Durchführung von Analysen in der täglichen Routine ermöglichen sollen.

Ein Verfahren zur Stabilisierung von wässrigen Coenzymen unter Verwendung von Chelatbildnern und Aziden ist aus JP 84/82398 bekannt. Nachteilig an diesem Verfahren ist jedoch, daß der Zusatz von Azid erforderlich ist, da Azid zum einen heute als Cancerogen eingestuft ist und zudem auf viele Enzyme eine inhibierende Wirkung hat. Darüber hinaus zeigt dieses Stabilisierungsverfahren bei höheren pH-Werten und Temperaturen deutliche Grenzen. Ferner ist seit längerem ein Verfahren zur Stabilisierung wäßriger Coenzymlösungen unter Zusatz eines organischen Lösungsmittels und einer inerten hygroskopischen Substanz beschrieben (US 4,310,624). Nachteilig bei dem Verfahren ist jedoch, daß es zum einen umständlich ist - insbesondere, da die hygroskopische Substanz anschließend wieder entfernt werden muß - und zum anderen den heutigen Stabilitätskriterien bei weitem nicht genügt.

Der vorliegenden Erfindung liegt somit die Aufgabe zugrunde, ein verbessertes ein Coenzym für wasserstoffübertragende Enzyme aufweisendes stabiles Flüssigreagenz. welches zur Bestimmung von Dehydrogenase-Aktivität bzw. entsprechender Substrate geeignet ist, zur Verfügung zu stellen.

Die Erfindung wird gelöst durch eine wässrige Lösung, welche ein Schwermetallsalz in einer Konzentration von ungefähr 1 bis 60 mM, bevorzugt in einer Konzentration von 2 bis 10 mM aufweist. Der pH-Wert entsprechender Arbeitslösungen liegt dabei im alkalischen Bereich, bevorzugt oberhalb von pH 8,5, besonders bevorzugt zwischen pH 8,5 und 10.0. Unter Arbeitslösungen wird erfindungsgemäß verstanden, daß die stabilisierte Coenzymlösung mit einer Lösung vermischt wird, die im wesentlichen aus einer im alkalischen Bereich puffernden Substanz besteht. Als vorteilhaft hat sich erwiesen, wenn das Verhältnis von Stabilisator zu Pufferlösung ca. 1:5 beträgt. Als Schwermetallsalz haben sich wasserlösliche Salze von Säuren mit den Schwermetallionen wie z.B. Cu²⁺ und/oder Co²⁺, aber auch die anderer Schwermetalle wie beispielsweise Eisen. Mangan, Zink, Nickel. Silber oder Palladium als geeignet erwiesen. Als Gegenion. d.h. sogenanntes Säureanion kommen die dem Fachmann bekannten Säurereste gängiger Säuren in Betracht, wie beispielsweise Sulfat, Phosphat, die Säurereste von Carbonsäuren und Halogenide. Insbesondere haben sich hier Kupfer bzw. Kobaltsulfat und/oder die entsprechenden Chloride als geeignet erwiesen.

Als Puffer sind solche Substanzen geeignet, die eine gute Pufferkapazität zwischen ca. pH 8.5 und 10,0 aufweisen. wie beispielsweise die sogenannten Good-Puffer (Tricine, Bicine, TAPS, AMPSO, CHES, CAPSO, AMP, CAPS), Carbonate von Alkalimetallionen, MEG, TRIS sowie Borat- oder Phosphatpuffer. Auch Mischungen der genannten Puffersubstanzen haben sich für die erfindungsgemäße Lösung als geeignet erwiesen. Als geeignet hat sich erwiesen, wenn die Pufferkonzentration zwischen 10 und 1000 mM, bevorzugt zwischen 400 und 600 mM beträgt.

Die Stabilität der Lösungen kann zudem weiter verbessert werden. wenn zusätzlich ein Komplexbildner, d.h. ein Ligand, welcher zwei oder mehr Koordinationsstellen aufweist, in der Lösung enthalten ist. Es haben sich hier sowohl zweizähnige Liganden wie z.B. Ethylendiamin und vier- bzw. mehrzähnige Liganden wie Ethylendiamin-N, N, N, N-tertraessigsäure (EDTA) bzw. entsprechende Salze, insbesondere das Dinatriumsalz, Kronenether oder Kryptanden als vorteilhaft erwiesen. Der Stabilisierungseffekt konnte insbesondere beobachtet werden, wenn der Komplexbildner in einem bestimmten Verhältnis zur Konzentration des Schwermetallsalzes zugegeben wird, wobei das jeweilige Ferner ist seit längerem ein Verfahren zur Stabilisierung wäßriger Coenzymlösungen unter Zusatz eines organischen Lösungsmittels und einer inerten hygroskopischen Substanz beschrieben (US 4,310,624). Nachteilig bei dem Verfahren ist jedoch, daß es zum einen umständlich ist - insbesondere, da die hygroskopische Substanz anschließend wieder entfernt werden muß - und zum anderen den heutigen Stabilitätskriterien bei weitem nicht genügt.

Schwermetallsalz im Überschuß vorliegt. Vorzugsweise beträgt das Verhältnis Metallion zu Komplexbildner 2 zu 1. Dies entspricht einer Konzentration des Komplexbildners von ungefähr 0.5 bis 30 mM, vorzugsweise von 1,0 bis 5,0 mM.

Als Coenzyme im Sinne der vorliegenden Erfindung kommen insbesondere NAD bzw. NADP, aber auch modifizierte Coenzyme, wie beispielsweise thioNAD(P) oder NHxDP (=Nicotinamidhypoxanthin-dinukleotidphosphat) in Betracht. Die Coenzyme können in einer Konzentration von ungefähr 1,0 bis 60 mM vorliegen, bevorzugt ist hier ein Bereich von 5,0 bis 15,0 mM.

Die stabilisierten Coenzym-Lösungen, d.h. sowohl mit einem Schwermetallsalz allein als auch zusammen mit dem Komplexbildner werden bevorzugt in Form von Lyophilisaten verwendet. Das gebrauchsfertige Reagenz ist darüber hinaus aber auch als Granulat, Pulvermischung sowie als wässrige Lösung über einen weiten Zeitraum hin stabil. So konnten bei Temperaturen von 2° bis 8°C innerhalb von 15 Monaten keinerlei Zersetzungserscheinungen des Reagenzes festgestellt werden. Unter Belastung, d.h. bei einer Temperatur von ca. 35°C für 2 Wochen und anschließende Behandlung bei ca. 42°C für einen Tag konnte gezeigt werden, daß die Lösung mit Schwermetallsalz bis zu ungefähr 60 mM unverändert, d.h. stabil bleibt.

Ein weiterer Gegenstand der Erfindung ist, ein Verfahren zur Bestimmung eines wasserstoffübertragenden Analyten bzw. einer entsprechenden Dehydrogenase in Gegenwart eines wasserstoffaufnehmenden Coenzyms, wobei das Coenzym in einer stabilisierten wässrigen alkalischen Lösung wie oben beschrieben vorliegt. Die Endkonzentration des Schwermetallsalzes im Test beträgt dabei vorzugsweise zwischen 0,1 und 1,0 mM; die Endkonzentration des gegebenenfalls zugesetzten Komplexbildner bevorzugt ca. 0.05 bis 5 mM.

Die Bestimmung erfolgt insbesondere in Proben biologischen Ursprungs wie beispielsweise Vollblut, Serum oder Plasma, oder Milch bzw. anderen menschlichen bzw. tierischen Quellen oder in pflanzlichen Extrakten. Die Probe kann mit physiologischer Kochsalzlösung aufbereitet werden. Als Kontrollwert dient in einem solchen Fall vorteilhafterweise eine 0,9% NaCl-Lösung.

Für den Fall, daß die Enzymaktivität einer Dehydrogenase wie beispielsweise Lactatdehydrogenase bestimmt werden soll, wird eine Substratlösung, beispielsweise eine Lactatlösung, in einer bei ca. pH 9,4 (37°C) puffernden Substanz(mischung) verwendet. Das Substrat kann dabei in den üblichen. dem Fachmann bekannten Konzentrationen eingesetzt werden, vorzugsweise in einem Bereich von 40 bis 80 mM.

Für die Bestimmung eines wasserstoffübertragenden Analyten wie beispielsweise Lactat, wird die jeweilige Dehydrogenase, wie beispielsweise LDH in einer zwischen pH 8,5 und 10.0 puffernden Substanz vorgelegt. In der Regel ist eine Menge an Dehydrogenase von ungefähr 70 bis 500 U/l, bevorzugt von 110 bis 220 U/l ausreichend. Die Bestimmung wird üblicherweise bei ca. 37°C durchgeführt.

Neben dem beispielhaft angeführten Lactat können in analoger Weise Glutamat bzw. Ammoniak, Alkohol, Glycerinaldehyd-3-phosphat, Glucose oder andere Parameter, welche durch eine geeignete Coenzymabhängige Dehydrogenase umgesetzt werden können, bestimmt werden. Entsprechendes gilt für die Bestimmung der Enzymaktivität solcher Dehydrogenasen.

Ein weiterer Gegenstand der Erfindung ist ein sogenannter Testkit zur Ausführung der Enzym- bzw. Analytbestimmung. Der Kit besteht im wesentlichen aus zwei Reagenzien. Das erste Reagenz beinhaltet - für den Fall der Bestimmung der Aktivität einer Dehydrogenase - einen wasserstoffübertragenden Analyten (Substrat) in einem geeigneten zwischen pH 8,5 und 10,0 puffernden System. Das zweite Reagenz weist ein Coenzym für wasserstoffübertragende Enzyme, wie beispielsweise NAD bzw. NADP und ein wasserlösliches Schwermetallsalz in einer Konzentration von ca. 1,0 und 60 mM. Das zweite Reagenz kann darüber hinaus einen Komplexbildner in einer Konzentration von 0,5 bis 30 mM enthalten. Ein analoges zweites Reagenz ist für den Fall der Analyt- bzw. Substratbestimmung, wie beispielsweise von Lactat, zu verwenden.

### Abkürzungen

AMP = 2-Amino-2-methyl-1-propanol
AMPSO = 3-[(1,1-Dimethyl-2-hydroylethyl)amino-2-hydroxypropansulfonsäure
Bicine = N,N-Bis[2-Hydroxyethyl] glycin
CAPS = 3-[Cyclohexylamino]-1-propansulfonsäure
CAPSO = 3-[Cyclohexylamino]-2-hydroxy-1-propansulfonsäure
CHES = 2-[N-Cyclohexylamino]ethansulfonsäure
MEG = N-Methylglucamin
TAPS = N-Tris[Hydroxymethyl]methyl-3-aminopropansulfonsäure
Tricine = N-Tris[Hydroxymethyl] methylglycerin
TRIS = 2-Amino-2-(hydroxymethyl)-3-propanol

Die folgenden Beispiele erläutern die Erfindung weiter:

### Beispiel 1

Reagenz 1: N-Methylglucamin 390 mmol/l pH 9,4 (37°C); Lithium-L-Lactat 60 mmol/l.
Reagenz 2: NAD(P) 60 mmol/l + Kupfer (II) Sulfat 5 mmol/l als Lyophilisat, Pulvermischung, Granulat oder wässrige Lösung.
Inkubationstemperatur: 37 ± 0,1°C; Meßwellenlänge 340 ± 2 nm; Schichtdicke 7 mm;
Vorinkubation: 5 Minuten; Lag phase: 2 Minuten; Meßzeit: 2 Minuten.

Reagenz 1 = 250 µl; Reagenz 2 = 50 µl; Probe 7 µl NACl-Lösung (0,9% w/v).

### a) Zusatz von 5 mmol/l Kupfer (II) Reagenz 2:

### Reagenzleerwert in mE/min

| | unbelastet | belastet (35°C 14 Tage und 42°C 1 Tag) |
|---|---|---|
| NAD (wässr. Lsg.) | 2,5 | 19,3 |
| NAD + 5 mmol/l Cu (II) (wässr. Lsg.) | 0,8 | 4,4 |

### b) Zusatz von 5 mmol/l Kupfer (II) + EDTA zu Reagenz 2:

### Reagenzleerwert in mE/min

| | unbelastet | belastet (35°C 14 Tage und 42°C 1 Tag) |
|---|---|---|
| NAD (wässr. Lsg.) | 2,5 | 19,3 |
| NAD + 5 mmol/l Cu + 2,5 mmol/l EDTA (wässr. Lsg.) | 0,8 | 5,0 |

### Beispiel 2

Es wurden die Reagenzien 1 und 2 aus Beispiel 1 mit den im folgenden beschriebenen geänderten Konzentratin an Kupfer (II)- bzw. Kobalt (II)-Suflat verwendet.

### a) Zusatz von 0-60 mmol/l Kupfer (II) Reagenz 2:

### Reagenzleerwert in mE/min

### NAD wässrige Lösung Zusatz von x mmol/l Cu (II)

| | unbelastet | belastet (1 Tag 42°C) |
|---|---|---|
| 0 mmol/l Cu (II) | 1,6 | 5,3 |
| 20 mmol/l Cu (II) | -0,2 | 0 |
| 40 mmol/l Cu (II) | 0,8 | 1,4 |
| 60 mmol/l Cu (II) | 2,1 | 2 |

### b) Zusatz von 3 mmol/l Kobalt (II) Reagenz 2:

### Reagenzleerwert in mE/min

| | unbelastet | belastet (1 Tag 42°C) |
|---|---|---|
| NAD (wässr. Lsg.) | 2,5 | 19,3 |
| NAD + 3 mmol/l Co (II) (wässr. Lsg.) | 0,6 | 18,5 |

### c) Zusatz von 5 mmol/l Kupfer (II) zu Reagenz 2 (NADP):

### 1. Unter Belastung (42°C)

### Reagenzleerwert in mE/min

| | unbelastet | belastet (1 Tag 42°C) |
|---|---|---|
| NADP (wässr. Lsg.) ohne CU (II) | -0,1 | 1,4 |
| NADP + 5 mmol/l Cu (wässr. Lsg.) | -0,8 | -0,6 |

### 2. Stabilität ohne Belastung (20°C)

| | Leerwert [me] 0 Monate | 9 Monate | 18 Monate * |
|---|---|---|---|
| NADP (wässr. Lsg.) ohne CU (II) | 2,3 | 11,9 | 21,5 |
| NADP + 5 mmol/l Cu (II) (wässr. Lsg.) | 0,7 | 2,2 | 3,7 |

| | | | |
|---|---|---|---|
| * extrapoliert | | | |

## Patentansprüche

1. Stabilisierte wässrige Lösung eines Coenzyms für wasserstoffübertragende Enzyme, dadurch gekennzeichnet, daß die Arbeitslösung einen pH-Wert von mindestens 8,5 aufweist und NAD, NADP oder ein entsprechendes Derivat in oxidierter oder reduzierter Form sowie 1,0 bis 60 mM eines wasserlöslichen Schwermetallsalzes enthält.

2. Stabilisierte Lösung nach Anspruch 1, dadurch gekennzeichnet, daß das Coenzym NAD oder NADP ist.

3. Stabilisierte Lösung nach Anspruch 1 oder 2, dadurch gekennzeichnet daß die Lösung zusätzlich einen Komplexbildner enthält.

4. Stabilisierte Lösung nach Anspruch 3, dadurch gekennzeichnet, daß das wasserlösliche Schwermetallsalz im Verhältnis zum Komplexbildner im Überschuß vorliegt.

5. Stabilisierte Lösung nach einem der Ansprüche 3 oder 4, dadurch gekennzeichnet, daß das Schwermetallsalz in einer Konzentration von ca. 2,0 bis 10,0 mM und der Komplexbildner in einer Konzentration von ca. 1,0 bis 5,0 mM vorhanden ist.

6. Stabilisierte Lösung nach einem der Ansprüche 1 bis 5, dadurch gekennzeichnet, daß das Schwermetallsalz aus Kupfer-, Kobalt-, Eisen-, Mangan-, Zink-, Nickel-, Silber- oder Palladiumkationen und aus einem Säureanion besteht und als der Komplexbildner Ethylendiamin, Ethylendiamin-N, N, N, N-tetraessigsäure bzw. das entsprechende Dinatriumsalz, Kronenether oder Kryptanden verwendet werden.

7. Verfahren zur Bestimmung eines wasserstoffübertragenden Analyten oder einer entsprechenden Dehydrogenase in Gegenwart eines wasserstoffaufnehmenden Coenzyms dadurch gekennzeichnet, daß das Coenzym in einer stabilisierten wässrigen alkalischen Lösung gemäß der Ansprüche 1 bis 6 zugesetzt wird.

8. Verfahren zur Bestimmung nach Anspruch 7. dadurch gekennzeichnet, daß der Analyt Lactat, Glutamat, Ammoniak, Alkohol, Glycerinaldehyd-3-phosphat oder Glucose in Gegenwart einer Lactat-, Glutamat-, Alkohol-, Glycerin-3-phosphat- oder Glucose-Dehydrogenase bestimmt wird.

9. Verfahren nach einem der Ansprüche 7 oder 8, dadurch gekennzeichnet, daß der pH-Wert in einem Bereich von 8,5 bis 10,0 liegt und die Endkonzentration des Schwermetallsalzes zwischen 0,1 und 1,0 mM beträgt.

10. Verfahren nach einem der Ansprüche 7 bis 9, dadurch gekennzeichnet, daß Lactat in Gegenwart von Lactatdehydrogenase bei einem pH-Wert von ungefähr 9,4 bestimmt wird.

11. Kit zur Bestimmung eines wasserstoffübertragenden Analyten in einer Probe bestehend aus folgenden Komponenten:
- einem Reagenz, welches eine Dehydrogenase in einem geeigneten zwischen pH 8,5 und 10,0 puffernden System enthält,
und
- einem Reagenz, welches ein Coenzym für wasserstoffübertragende Enzyme und ein wasserlösliches Schwermetallsalz in einer Konzentration zwischen 1,0 und 60 mM enthält.

12. Kit zur Bestimmung der Enzymaktivität einer Dehydrogenase in einer Probe bestehend aus folgenden Komponenten:
- einem Reagenz, welches einen wasserstoffübertragenden Analyten in einem geeigneten zwischen pH 8,5 und 10,0 puffernden System enthält,
und
- einem Reagenz, welches ein Coenzym für wasserstoffübertragende Enzyme und ein wasserlösliches Schwermetallsalz in einer Konzentration zwischen 1,0 und 60 mM enthält.

13. Kit nach Anspruch 11 oder 12, dadurch gekennzeichnet, daß das zweite Reagenz zusätzlich einen Komplexbildner in einer Konzentration von 0,5 bis 30 mM enthält.

14. Kit nach Anspruch 11, 12 oder 13, dadurch gekennzeichnet, daß es sich bei dem Coenzym um NAD bzw. NADP oder ein entsprechendes Derivat in oxidierter Form oder reduzierter Form handelt.

## Claims

1. Stabilized aqueous solution of a coenzyme for hydrogen-transferring enzymes, wherein the working solution has a pH value of at least 8.5 and contains NAD, NADP or an appropriate derivative in an oxidized or reduced form as well as 1.0 to 60 mM of a water-soluble heavy metal salt.

2. Stabilized solution as claimed in claim 1, wherein the coenzyme is NAD or NADP.

3. Stabilized solution as claimed in claim 1 or 2, wherein the solution additionally contains a complexing agent.

4. Stabilized solution as claimed in claim 3, wherein the water-soluble heavy metal salt is present in an excess relative to the complexing agent.

5. Stabilized solution as claimed in one of the claims 3 or 4, wherein the heavy metal salt is present at a concentration of ca. 2.0 to 10.0 mM and the complexing agent is present at a concentration of ca. 1.0 to 5.0 mM.

6. Stabilized solution as claimed in one of the claims 1 to 5, wherein the heavy metal salt is composed of copper, cobalt, iron, manganese, zinc, nickel, silver or palladium cations and of an acid anion and ethylenediamine, ethylenediamine-N,N,N,N-tetraacetic acid or the corresponding disodium salt, crown ethers or cryptands are used as the complexing agent.

7. Method for the determination of a hydrogen-transferring analyte or of a corresponding dehydrogenase in the presence of a hydrogen-accepting coenzyme, wherein the coenzyme is added in a stabilized aqueous alkaline solution as claimed in claims 1 to 6.

8. Method for the determination as claimed in claim 7, wherein the analyte lactate, glutamate, ammonia, alcohol, glyceraldehyde-3-phosphate or glucose is determined in the presence of a lactate, glutamate, alcohol, glycerol-3-phosphate or glucose dehydrogenase.

9. Method as claimed in one of the claims 7 or 8, wherein the pH value is in the range of 8.5 to 10.0 and the final concentration of the heavy metal salt is between 0.1 and 1.0 mM.

10. Method as claimed in one of the claims 7 to 9, wherein lactate is determined in the presence of lactate dehydrogenase at a pH value of about 9.4.

11. Kit for the determination of a hydrogen-transferring analyte in a sample comprising the following components:
- a reagent which contains a dehydrogenase in a suitable system buffering between pH 8.5 and 10.0
and
- a reagent which contains a coenzyme for hydrogen-transferring enzymes and a water-soluble heavy metal salt at a concentration between 1.0 and 60 mM.

12. Kit for the determination of the enzyme activity of a dehydrogenase in a sample comprising the following components:
- a reagent which contains a hydrogen-transferring analyte in a suitable system buffering between pH 8.5 and 10.0
and
- a reagent which contains a coenzyme for hydrogen-transferring enzymes and a water-soluble heavy metal salt at a concentration between 1.0 and 60 mM.

13. Kit as claimed in claim 11 or 12, wherein the second reagent additionally contains a complexing agent at a concentration of 0.5 to 30 mM.

14. Kit as claimed in claim 11, 12 or 13, wherein the coenzyme is NAD or NADP or a corresponding derivative in an oxidized form or reduced form.

## Revendications

1. Solution aqueuse stabilisée d'un coenzyme pour enzyme transférant l'hydrogène, caractérisée en ce que la solution de travail présente une valeur de pH d'au moins 8,5 et contient du NAD, du NADP ou un dérivé correspondant sous forme oxydée ou réduite, ainsi que de 1,0 à 60 mM d'un sel de métal lourd soluble dans l'eau.

2. Solution stabilisée selon la revendication 1, caractérisée en ce que le coenzyme est le NAD ou le NADP.

3. Solution stabilisée selon la revendication 1 ou 2. caractérisée en ce que la solution contient en outre un agent complexant.

4. Solution stabilisée selon la revendication 3, caractérisée en ce que le sel de métal lourd soluble dans l'eau est présent en excès par rapport à l'agent complexant.

5. Solution stabilisée selon l'une des revendications 3 ou 4, caractérisée en ce que le sel de métal lourd est présent à une concentration d'environ 2,0 à 10,0 mM et l'agent complexant à une concentration d'environ 1,0 à 5,0 mM.

6. Solution stabilisée selon l'une des revendications 1 à 5, caractérisée en ce que le sel de métal lourd est constitué de cations cuivre, cobalt, fer, manganèse, zinc, nickel, argent ou palladium et d'un anion acide et que l'on utilise comme agent complexant l'éthylènediamine, I'éthylènediamine-N,N,N,N-tétraacétique ou le sel bisodique correspondant, les éthers couronnes ou les agents chélatant correspondants.

7. Procédé pour déterminer un analyte transférant l'hydrogène ou une déshydrogénase correspondante en présence d'un coenzyme absorbant l'hydrogène, caractérisé en ce que le coenzyme est ajouté à une solution aqueuse alcaline stabilisée selon l'une des revendications 1 à 6.

8. Procédé de détermination selon la revendication 7, caractérisé en ce que l'analyte lactate, glutamate, ammoniac, alcool, triphosphate de glycérine aldéhyde ou glucose est déterminé en présence d'un lactate-, glutamate-, alcool-, glycérine-3-phosphate ou glucose-déshydrogénase.

9. Procédé selon l'une des revendications 7 ou 8, caractérisé en ce que la valeur du pH est comprise dans l'intervalle de 8,5 à 10,0 et que la concentration finale du sel de métal lourd se situe entre 0,1 et 1,0 mM.

10. Procédé selon l'une des revendications 7 à 9, caractérisé en ce que le lactate est déterminé en présence de lactate déshydrogénase à une valeur de pH d'environ 9,4.

11. Trousse pour déterminer un analyte transférant l'hydrogène dans un échantillon constitué des composants suivants:
- un réactif qui contient une déshydrogénase dans un système tampon approprié entre les pH de 8,5 et 10,0,
et
- un réactif qui contient un coenzyme pour enzymes transférant l'hydrogène et un sel de métal lourd soluble dans l'eau à une concentration comprise entre 1,0 et 60 mM.

12. Trousse pour déterminer l'activité enzymatique d'une déshydrogénase dans un échantillon constitué des composants suivants:
- un réactif qui contient un analyte transférant l'hydrogène dans un système tampon approprié entre pH 8,5 et 10,0,
et
- un réactif qui contient un coenzyme pour enzymes transférant l'hydrogène et un sel de métal lourd soluble dans l'eau à une concentration comprise entre 1,0 et 60 mM.

13. Trousse selon les revendications 11 ou 12, caractérisée en ce que le deuxième réactif contient en outre un agent complexant à une concentration de 0,5 à 30 mM.

14. Trousse selon la revendication 11, 12 ou 13, caractérisée en ce que le coenzyme est du NAD ou du NADP ou un dérivé correspondant sous forme oxydée ou réduite.
